# EUROPÄISCHE PATENTSCHRIFT

(11) **EP 1 819 749 B1**
(45) Veröffentlichungstag und Bekanntmachung des Hinweises auf die Patenterteilung: **27.10.2010**
(21) Anmeldenummer: 05817915.1
(22) Anmeldetag: 06.12.2005
(51) Int. Cl.: C08G 61/10, C08G 61/02, C07C 43/225, C07C 211/56, C07C 25/22

(54) **TEILKONJUGIERTE POLYMERE, DEREN DARSTELLUNG UND VERWENDUNG**
PARTIALLY CONJUGATED POLYMERS, THEIR REPRESENTATION AND THEIR USE
POLYMERES PARTIELLEMENT CONJUGUES, LEUR REPRESENTATION ET LEUR UTILISATION

(30) Priorität: 06.12.2004 EP 04028865
(43) Veröffentlichungstag der Anmeldung: 22.08.2007
(73) Patentinhaber: Merck Patent GmbH, 64293 Darmstadt (DE)
(72) Erfinder: HEUN, Susanne, 65812 Bad Soden (DE); PARHAM, Amir, 65929 Frankfurt (DE); LUDEMANN, Aurelie, 60322 Frankfurt (DE)
(86) Internationale Anmeldenummer: PCT/EP2005/013043
(87) Internationale Veröffentlichungsnummer: WO 2006/061181

(56) Entgegenhaltungen:
- WO-A-03/020790
- DE-A1- 10 241 814
- US-B1- 6 361 884

## Beschreibung

Seit einigen Jahren läuft eine breit angelegte Forschung zur Kommerzialisierung von Anzeige- und Beleuchtungselementen auf Basis polymerer (organischer) Leuchtdioden (PLEDs). Ausgelöst wurde diese Entwicklung durch die Grundlagenentwicklungen, welche in WO 90/13148 offenbart sind. Seit kurzem sind auch erste, einfache Produkte (kleine Anzeigen in einem Rasierapparat und einem Mobiltelefon der Fa. PHILIPS N.V.) auf dem Markt erhältlich. Allerdings sind immer noch deutliche Verbesserungen der verwendeten Materialien nötig, um diese Displays zu einer echten Konkurrenz zu den derzeit marktbeherrschenden Flüssigkristallanzeigen (LCD) zu machen. Dabei ist es für die Erzeugung aller drei Emissionsfarben nötig, bestimmte Comonomere in die entsprechenden Polymere einzupolymerisieren (vgl. z. B. WO 00/46321, WO 03/020790 und WO 02/077060). So ist dann in der Regel, ausgehend von einem blau emittierenden Grundpolymer ("backbone"), die Erzeugung der beiden anderen Primärfarben Rot und Grün möglich.

Die konjugierten Polymere gemäß dem Stand der Technik zeigen zum Teil schon gute Eigenschaften in der Anwendung in PLEDs. Trotz der bereits erzielten Fortschritte entsprechen diese Polymere allerdings noch nicht den Anforderungen, die an sie für hochwertige Anwendungen gestellt werden. Insbesondere ist die Lebensdauer der grün und vor allem der blau emittierenden Polymere für viele Anwendungen noch nicht ausreichend.

Wir vermuten, dass die elektronischen Eigenschaften der konjugierten Polymere noch nicht optimal für einen ausbalancierten Ladungstransport in der elektronischen Vorrichtung sind. Dadurch ist teilweise der Elektronen- bzw. der Lochstrom in der Vorrichtung zu groß, und es kann sich kein ausbalanciertes Ladungsgleichgewicht einstellen. Ein weiterer Nachteil ist die statistische breite Verteilung effektiver Konjugationslängen im Polymer, die zu Polymerabschnitten undefinierter Länge und relativ breitbandiger Emission und damit geringer Farbreinheit führt.

Trotz der bereits erzielten Fortschritte gibt es immer noch einen erheblichen Bedarf an Verbesserung für entsprechende Materialien. V. a. auf folgenden Feldern ist noch ein deutlicher Verbesserungsbedarf zu sehen:
- Die Effizienz bei hohen Leuchtdichten ist bei allen Farben noch verbesserungsbedürftig. Dies ist vor allem für die Verwendung in so genannten Passiv-Matrix (PM) gesteuerten Displays von entscheidender Bedeutung: In diesen PM-Displays, wird jeder einzelne Bildpunkt (Pixel) nur einen Bruchteil der Zeit angesteuert (dieser Bruchteil wird mit der sogenannten Multiplex-Rate (MUX) bezeichnet). Ein MUX-64 bzw. MUX-128 Display bedeutet, dass jeder einzelne Pixel nur 1/64 bzw. 1/128 der Gesamtzeit angesteuert wird. Um trotzdem die gewünschte Helligkeit zu erhalten, muss in dieser kurzen Zeit der jeweilige Pixel mit demselben Faktor (also in diesen Fällen 64 bzw. 128) heller leuchten, als dies für die gewünschte Helligkeit eigentlich nötig wäre. Will man also beispielsweise eine Pixel mit einer Durchschnittshelligkeit von 200 cd/m² betreiben, benötigt man kurzfristig 12800 bzw. gar 25600 cd/m². Durch die Trägheit des menschlichen Auges erhält der Betrachter dann - bei geeigneter Ansteuerung - den Eindruck des durchschnittlichen Helligkeitswertes. Problem bei dieser Ansteuerung ist nun, dass die bisher benutzten Polymere eine starke Abhängigkeit der Effizienz von der benötigten Helligkeit aufweisen. Daraus resultieren für PM-angesteuerte Displays große Schwierigkeiten.
- Die operative Lebensdauer, v. a. bei BLAU emittierenden Polymeren und bei PM-Ansteuerung, bedarf immer noch einer deutlichen Verbesserung, um diese für marktgerechte Anwendungen einsetzen zu können.

In WO 97/20877 und in EP 0882082 werden teilkonjugierte Spirobifluoren-Polymere beschrieben, die über die 2,2'-Positionen der Spirobifluoren-Einheiten verknüpft sind. Als Vorteile dieser Polymere wird vor allem die exzellente Verarbeitbarkeit aufgeführt. Jedoch wird zu den elektronischen Eigenschaften nur berichtet, dass bei Anlegen einer ausreichend hohen Spannung Elektrolumineszenz beobachtet wird, ohne eine Aussage über Spannung, Effizienz und Lebensdauer zu treffen. Auch in einigen Publikationen wird die Verwendung von 2,2'-verknüpften Spirobifluoren-Einheiten beschrieben, z. B. von R. D. Miller et al. (Polymer Preprints 2002, 43, 116-117), F.-I. Wu et al. (J. Mater. Chem. 2002, 12, 2893-2897) und B. Huang et al. (Chem. Lett. 2004, 33, 1376-1377). In keiner dieser Publikationen lässt sich jedoch eine besonders gute Eignung dieser Polymere für organische elektrolumineszente Vorrichtungen erkennen.

Es wurde nun überraschend gefunden, dass teilkonjugierte Polymere, die über die beiden nicht miteinander konjugierten Hälften von Spirobifluoren, jedoch über andere als die 2,2'-Positionen, verknüpft sind, sehr gute und den Stand der Technik übertreffende Eigenschaften aufweisen. Diese Polymere zeigen insbesondere bessere Eigenschaften in der Elektrolumineszenz als vergleichbare Polymere, die über die 2,2'-Positionen des Spirobifluorens verknüpft sind. Dies bezieht sich insbesondere auf die Lebensdauer bei PM-Ansteuerung, aber auch auf die Emissionsfarbe, die Strom-Spannungskurven und die Effizienz der Polymere. Dies ist ein überraschendes und unerwartetes Ergebnis. Diese Polymere und deren Verwendung in organischen elektronischen Vorrichtungen sind daher Gegenstand der vorliegenden Erfindung.

Gegenstand der Erfindung sind Polymere, enthaltend mindestens 1 mol%, bevorzugt mindestens 5 mol%, besonders bevorzugt mindestens 10 mol% einer ersten Wiederholeinheit gemäß Formel (1), 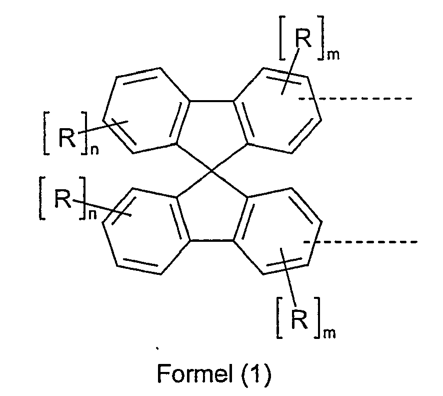 wobei die verwendeten Symbole und Indizes die folgende Bedeutung besitzen:
- R: ist bei jedem Auftreten gleich oder verschieden F, Cl, Br, I, CN, NO₂, OH, N(R¹)₂, Si(R¹)₃, B(R¹)₂, eine geradkettige Alkyl-, Alkoxy-oderThioalkoxygruppe mit 1 bis 40 C-Atomen oder eine verzweigte oder cyclische Alkyl-, Alkoxy- oder Thioalkoxygruppe mit 3 bis 40 C-Atomen, in der auch ein oder mehrere nicht benachbare C-Atome durch -CR¹=CR¹, -C≡C-, -NR¹-, -O-, -S-, -CO-O-, C=O, P(=O)R¹, SO, SO₂ oder -O-CO-O- ersetzt sein können und wobei auch ein oder mehrere H-Atome durch Fluor ersetzt sein können, oder ein aromatisches oder heteroaromatisches Ringsystem mit 5 bis 40 aromatischen Ringatomen, welches auch durch einen oder mehrere nicht-aromatische Reste R substituiert sein kann; oder eine Kombination aus 2, 3, 4 oder 5 der oben genannten Gruppen; dabei können auch zwei oder mehrere der Reste R miteinander ein aliphatisches oder aromatisches, mono- oder polycyclisches Ringsystem bilden;
- R¹: ist bei jedem Auftreten gleich oder verschieden H oder ein aliphatischer oder aromatischer Kohlenwasserstoffrest mit 1 bis 20 C-Atomen;
- n: ist bei jedem Auftreten gleich oder verschieden 0, 1, 2, 3 oder 4;
- m: ist bei jedem Auftreten gleich oder verschieden 0, 1, 2 oder 3;
die gestrichelten Bindungen deuten dabei die Verknüpfung im Polymer an; mit der Maßgabe, dass die Verknüpfung nicht über die 2,2'-Positionen erfolgt; und enthaltend mindestens 1 mol% einer zweiten Wiederholeinheit, die entweder gleich einer Wiederholeinheit gemäß Formel (1) ist oder verschieden ist.

Die Positionen am Spirobifluoren sind im Folgenden der Übersichtlichkeit halber aufgeführt:

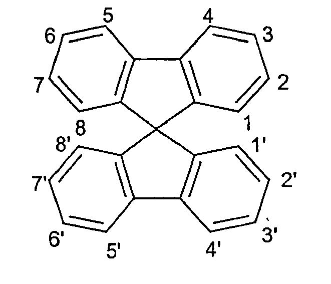

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl; n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 40 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Auch wenn dies aus der Beschreibung hervorgeht, sei hier nochmals explizit darauf verwiesen, dass die Struktureinheiten gemäß Formel (1) unsymmetrisch substituiert sein können, d. h. dass in einer Einheit unterschiedliche Substituenten R vorhanden sein können, bzw. diese auch an unterschiedlichen Positionen gebunden sein können.

Unter einem aromatischen bzw. heteroaromatischen Ringsystem im Sinne dieser Erfindung soll ein System verstanden werden, das nicht notwendigerweise nur aromatische bzw. heteroaromatische Gruppen enthält, sondern in dem auch mehrere aromatische bzw. heteroaromatische Gruppen durch eine kurze nicht-aromatische Einheit (< 10 % der von H verschiedenen Atome, bevorzugt < 5 % der von H verschiedenen Atome), wie beispielsweise sp³-hybridisierter C, O, N, etc., unterbrochen sein können. So sollen also beispielsweise auch Systeme wie 9,9'-Spirobifluoren, 9,9-Diarylfluoren, Triarylamin, etc. als aromatische Systeme im Sinne dieser Erfindung verstanden werden.

Einheiten gemäß Formel (1) liegen in zwei enantiomeren Formen vor. Dabei ist es gleichermaßen erfindungsgemäß, das Racemat, also die 1 : 1 Mischung der beiden Enantiomeren, oder eines der beiden Enantiomeren in angereicherter oder isolierter Form zu verwenden.

Bevorzugt sind Einheiten gemäß Formel (1), in denen für die Symbole und Indizes gilt:
- R: ist bei jedem Auftreten gleich oder verschieden F, CN, NO₂, OH, N(R¹)₂, eine geradkettige Alkyl-, Alkoxy- oder Thioalkoxygruppe mit 1 bis 20 C-Atomen oder eine verzweigte oder cyclische Alkyl-, Alkoxy- oder Thioalkoxygruppe mit 3 bis 40 C-Atomen, in der auch ein oder mehrere nicht benachbarte C-Atome durch -CR¹=CR¹-, -C≡C-, -NR¹-, -O-, -S-, C=O oder P(=O)R¹ ersetzt sein können und wobei auch ein oder mehrere H-Atome durch Fluor ersetzt sein können, oder ein aromatisches oder heteroaromatisches Ringsystem mit 5 bis 30 aromatischen Ringatomen, welches auch durch einen oder mehrere nicht- aromatische Reste R substituiert sein kann; oder eine Kombination aus 2, 3 oder 4 der oben genannten Gruppen; dabei können auch zwei oder mehrere der Reste R miteinander ein aliphatisches oder aromatisches, mono- oder polycyclisches Ringsystem bilden;
- R¹: ist wie oben beschrieben;
- n: ist bei jedem Auftreten gleich oder verschieden 0, 1, 2 oder 3;
- m: ist bei jedem Auftreten gleich oder verschieden 0, 1 oder 2.

Besonders bevorzugt sind Einheiten gemäß Formel (1), in denen für die Symbole und Indizes gilt:
- R: ist bei jedem Auftreten gleich oder verschieden F, N(R¹)₂, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, in der jeweils auch ein oder mehrere nicht benachbarte C-Atome durch -CR¹=CR¹-, -C≡C-, -O- oder C=O ersetzt sein können und wobei auch ein oder mehrere H-Atome durch Fluor ersetzt sein können, oder ein aromatisches oder heteroaromatisches Ringsystem mit 5 bis 20 aromatischen Ringatomen, welches auch durch einen oder mehrere nicht-aromatische Reste R substituiert sein kann; oder eine Kombination aus 2 oder 3 der oben genannten Gruppen; dabei können auch zwei oder mehrere der Reste R miteinander ein aliphatisches oder aromatisches, mono- oder polycyclisches Ringsystem bilden;
- R¹: ist wie oben beschrieben;
- n: ist bei jedem Auftreten gleich oder verschieden 0, 1 oder 2;
- m: ist bei jedem Auftreten gleich oder verschieden 0 oder 1.

Weiterhin bevorzugt sind Einheiten gemäß Formel (1), die an den beiden Spiro-Molekülhälften symmetrisch substituiert sind. Diese Bevorzugung ist durch die leichtere synthetische Zugänglichkeit der Monomere zu begründen.

Erfindungsgemäß sind verschiedene Verknüpfungen der Einheiten gemäß Formel (1) im Polymer möglich. Bevorzugt ist, wenn die Verknüpfung über gleiche Positionen an den beiden Spiro-Molekülhälften erfolgt. Diese Bevorzugung ergibt sich wiederum aus der leichteren synthetischen Zugänglichkeit der entsprechenden Monomere. Eine bevorzugte Ausführungsform der Erfindung ist daher die Verknüpfung über die 1,1'-Positionen, über die 3,3'-Positionen oder über die 4,4'-Positionen. Eine besonders bevorzugte Ausführungsform der Erfindung ist die Verknüpfung über die 1,1'-Positionen oder über die 4,4'-Positionen. Ganz besonders bevorzugt ist die Verknüpfung über die 4,4'-Positionen.

Aus der Art der Verknüpfung folgt, dass die Einheiten gemäß Formel (1) in die Hauptkette des Polymers gebunden sind und dass die Einheiten gemäß Formel (1) zu einem Knick in der Polymerkette führen, da zwischen den beiden Verknüpfungen zum Polymer das sp³-hybridisierte Spiro-Kohlenstoffatom liegt, das für die orthogonale Verknüpfung der beiden Spiro-Molekülhälften verantwortlich ist.

Die erfindungsgemäßen Polymere können teilkonjugiert oder nicht-konjugiert sein. Vollständig konjugierte Polymere sind hier nicht möglich, da die Einheiten gemäß Formel (1) immer eine Konjugationsunterbrechung darstellen. In einer bevorzugten Ausführungsform der Erfindung handelt es sich um ein teilkonjugiertes Polymer. Besonders bevorzugt enthält das Polymer außer Einheiten gemäß Formel (1) keine weiteren Einheiten, die die Konjugation des Polymers unterbrechen.

Unter einem teilkonjugierten Polymer im Sinne dieser Erfindung soll ein Polymer verstanden werden, in dem längere konjugierte Abschnitte in der Hauptkette durch nicht-konjugierte Abschnitte unterbrochen sind, welche, wie oben erwähnt, bevorzugt durch Einheiten gemäß Formel (1) erzeugt werden. Diese konjugierten Abschnitte werden durch sp²-hybridisierte (oder auch sp-hybridisierte) Kohlenstoffatome erzeugt, die auch durch entsprechende Heteroatome ersetzt sein können, was im einfachsten Fall abwechselndes Vorliegen von Doppel- (bzw. auch Dreifach-) und Einfachbindungen bedeutet. Des Weiteren wird in diesem Anmeldetext ebenfalls als konjugiert bezeichnet, wenn sich in der Hauptkette beispielsweise Arylamineinheiten und/oder bestimmte Heterocyclen (d. h. Konjugation über N-, O- oder S-Atome) und/oder metallorganische Komplexe (d. h. Konjugation über das Metallatom) befinden. Hingegen würden Einheiten wie beispielsweise einfache Alkylenbrücken, (Thio)Ether-, Ester-, Amid- oder Imidverknüpfungen als nicht-konjugierte Segmente definiert.

Die erfindungsgemäßen Polymere enthalten neben Einheiten gemäß Formel (1) bevorzugt noch weitere Strukturelemente, die verschieden von Einheiten gemäß Formel (1) sind, und sind somit als Copolymere zu bezeichnen. Bevorzugt sind die weiteren Strukturelemente konjugiert. Hier sei vor allem auch auf die relativ umfangreichen Auflistungen in WO 02/077060, in WO 05/014689 und die darin aufgeführten Zitate verwiesen. Diese weiteren Struktureinheiten können beispielsweise aus den im Folgenden beschriebenen Klassen stammen:
Gruppe 1: Aromatische Einheiten, welche üblicherweise das Polymer-Grundgerüst
   darstellen:
   Einheiten dieser Gruppe sind aromatische, carbocyclische Strukturen mit 6 bis 40 C-Atomen, die substituiert oder unsubstituiert sein können, wobei als Substituenten die oben genannten Reste R in Frage kommen. Hier kommen Fluoren-Derivate (z. B. EP 0842208, WO 99/54385, WO 00/22027, WO 00/22026, WO 00/46321) in Betracht. Des Weiteren sind auch Spirobifluoren-Derivate (z. B. EP 0707020, EP 0894107, WO 03/020790) eine Möglichkeit. Auch Polymere, die eine Kombination der beiden erstgenannten Monomer-Einheiten enthalten (WO 02/077060), wurden bereits vorgeschlagen. In WO 05/014689 sind Dihydrophenanthren-Derivate beschrieben. Weiterhin kommen cis- oder trans-Indenofluoren-Derivate (z. B. WO 04/041901, WO 04/113412) in Frage, aber auch 1,4-Phenylen-Derivate, 4,4'-Biphenylylen-Derivate, 4,4"-Terphenylylen-Derivate, 2,7-oder 3,6-Phenanthren-Derivate (z. B. DE 0102004020298.2), Dihydropyren- oder Tetrahydropyren-Derivate und weitere nicht explizit aufgeführte aromatische Strukturen. Einheiten aus Gruppe 1 sind also bevorzugt ausgewählt aus der Gruppe der Fluoren-Derivate, der Spirobifluoren-Derivate, der Dihydrophenanthren-Derivate, der cis- oder trans-Indenofluoren-Derivate, der 1,4-Phenylen-Derivate, der 4,4'-Biphenylen-Derivate, der 4,4"-Terphenylen-Derivate, der 2,7- oder 3,6-Phenanthren-Derivate, der Dihydropyren- oder der Tetrahydropyren-Derivate. Besonders bevorzugte Einheiten aus dieser Gruppe sind ausgewählt aus Spirobifluoren, Fluoren, Dihydrophenanthren, cis-Indenofluoren, trans-Indenofluoren und 2,7-Phenanthren, die durch R substituiert oder unsubstituiert sein können.
Gruppe 2: Einheiten, welche die Morphologie bzw. die Emissionsfarbe verändern:
   Es sind auch Strukturelemente möglich, die die Morphologie, aber auch die Emissionsfarbe der resultierenden Polymere beeinflussen können. Bevorzugt sind diese dabei ausgewählt aus der Gruppe der durch R substituierten oder unsubstituierten kondensierten aromatischen Strukturen mit 6 bis 40 C-Atomen oder auch Tolan-, Stilben- oder Bisstyrylarylenderivaten, wie z. B. 1,4-Naphthylen-, 1,4-oder 9,10-Anthrylen-, 1,6- oder 2;7- oder 4,9-Pyrenylen-, 3,9- oder 3,10-Perylenylen-, 4,4'-Bi-1,1'-naphthylylen-, 4,4'-Tolanylen-, 4,4'-Stilbenylen- oder 4,4"-Bisstyrylarylenderivate.
Gruppe 3: Einheiten, welche die Lochinjektions- und/oder -transporteigenschaften
   der Polymere erhöhen:
   Dies sind im Allgemeinen aromatische Amine oder Phosphine oder elektronenreiche Heterocyclen und sind bevorzugt ausgewählt aus der Gruppe der durch R substituierten oder unsubstituierten Triarylamine, Benzidine, N,N,N',N'-Tetraaryl-para-phenylendiamine, Triarylphosphine, Phenothiazine, Phenoxazine, Dihydrophenazine, Thianthrene, Dibenzo-p-dioxine, Phenoxathiine, Carbazole, Azulene, Thiophene, Pyrrole, Furane und weiteren O-, S- oder N-haltigen Heterocyclen mit hoch liegendem HOMO (HOMO = höchstes besetztes Molekülorbital). Diese Einheiten können in die Hauptkette oder in die Seitenkette des Polymers eingebaut werden. Je nach Struktur ist auch das Polymergrundgerüst in der Lage, ausreichend gut Löcher zu leiten, so dass nicht zwangsläufig Einheiten aus Gruppe 3 anwesend sein müssen.
Gruppe 4: Einheiten, welche die Elektroneninjektions- und/oder -transporteigenschaften der Polymere erhöhen:
   Dies sind im Allgemeinen elektronenarme Aromaten oder Heterocyclen und sind bevorzugt ausgewählt aus der Gruppe der durch R substituierten oder unsubstituierten Pyridine, Pyrimidine, Pyridazine, Pyrazine, Triazine, Oxadiazole, Chinoline, Chinoxaline oder Phenazine, aber auch Verbindungen wie Triarylborane und weiteren O-, S- oder N-haltigen Heterocyclen mit niedrig liegendem LUMO (LUMO = niedrigstes unbesetztes Molekülorbital). Je nach Struktur ist auch das Polymergrundgerüst in der Lage, ausreichend gut Elektronen zu leiten, so dass nicht zwangsläufig Einheiten aus Gruppe 4 vorhanden sein müssen.
Gruppe 5: Einheiten, die Kombinationen von Einzeleinheiten der Gruppe 3 und Gruppe 4 aufweisen:
   Es kann auch bevorzugt sein, wenn in den erfindungsgemäßen Polymeren Einheiten enthalten sind, in denen Strukturen, welche die Lochmobilität und welche die Elektronenmobilität erhöhen, direkt aneinander gebunden sind, also Strukturen aus den oben genannten Gruppen 3 und 4. Viele dieser Einheiten verschieben die Emissionsfarbe ins Grüne, Gelbe oder Rote; ihre Verwendung eignet sich also beispielsweise für die Erzeugung anderer Emissionsfarben aus ursprünglich blau emittierenden Polymeren.
Gruppe 6: Einheiten, die aus dem Triplett-Zustand Licht emittieren Struktureinheiten aus dieser Gruppe können auch bei Raumtemperatur mit hoher Effizienz aus dem Triplettzustand Licht emittieren und zeigen also Elektrophosphoreszenz statt Elektrofluoreszenz. Hierfür eignen sich zunächst Verbindungen, welche Schweratome mit einer Ordnungszahl von mehr als 36 enthalten. Besonders geeignet sind Verbindungen, welche d- oder f-Übergangsmetalle enthalten, die diese Bedingung erfüllen. Ganz besonders bevorzugt sind Struktureinheiten, welche Elemente der Gruppe 8 bis 10 (Ru, Os, Rh, Ir, Pd, Pt) enthalten. Diese Metallkomplexe können in die Hauptkette und/oder in die Seitenkette des Polymers gebunden werden. Als geeignet hat sich auch der Einbau derartiger Metallkomplexe an Verzweigungspunkte im Polymer erwiesen, wie beispielsweise in DE 102004032527.8 beschrieben. Wenn Einheiten aus Gruppe 6 vorhanden sind, kann es bevorzugt sein, wenn gleichzeitig auch Einheiten aus Gruppe 7 vorhanden sind. Auch auch ohne derartige Einheiten aus Gruppe 7 können mit Triplett-Emittern sehr gute Ergebnisse und sehr hohe Effizienzen erreicht werden.
Gruppe 7: Einheiten, welche den Transfer vom Singulett- zum Triplett-Zustand unterstützen
   Für den Einsatz von Triplett-Emittern kann es bevorzugt sein, unterstützend weitere Strukturelemente einzusetzen, welche den Übergang vom Singulett- zum Triplett-Zustand und damit die Elektrophosphoreszenzeigenschaften verbessern. Hierfür kommen beispielsweise Carbazoleinheiten in Frage, wie in WO 04/070772 und WO 04/113468 beschrieben, aber beispielsweise auch Keto-, Phosphinoxid-, Sulfoxid- oder Sulfon-Einheiten, wie in WO 05/040302 beschrieben, oder Silaneinheiten, wie in der nicht offen gelegten Anmeldung DE 102004023278.4 beschrieben. Die Einheiten sind also bevorzugt ausgewählt aus der Gruppe der Carbazoleinheiten, der überbrückten Carbazoleinheiten, der Keto-, Phosphinoxid-, Sulfoxid- oder Sulfon-Einheiten und der Silaneinheiten.

Bevorzugt sind Polymere, die neben Struktureinheiten gemäß Formel (1) zusätzlich noch eine oder mehrere Einheiten ausgewählt aus den Gruppen 1 bis 7 enthalten. Dabei kann es auch vorteilhaft sein, wenn gleichzeitig mehr als eine Struktureinheit aus einer der Gruppen 1 bis 7 vorliegt. Besonders bevorzugt sind Polymere, die neben Einheiten, gemäß Formel (1) noch Einheiten aus der Gruppe 1 enthalten, ganz besonders bevorzugt mindestens 30 mol% dieser Einheiten.

Besonders bevorzugt sind weiterhin Polymere, die neben Einheiten gemäß Formel (1) noch Einheiten aus der Gruppe 2 und/oder 3 enthalten, ganz besonders bevorzugt mindestens 5 mol% dieser Einheiten.

Die nötige Löslichkeit der Polymere wird v. a. durch die Substituenten an den verschiedenen Wiederholeinheiten gewährleistet, sowohl den Substituenten R und R¹ an Einheiten gemäß Formel (1), wie auch durch Substituenten R an den anderen Wiederholeinheiten.

Bevorzugt ist ein Anteil von 1 - 100 mol% Einheiten gemäß Formel (1). Besonders bevorzugt ist ein Anteil von 5 - 70 mol% Einheiten gemäß Formel (1), ganz besonders bevorzugt ein Anteil von 10 - 50 mol%.

Die erfindungsgemäßen Polymere sind entweder Homopolymere aus Einheiten gemäß Formel (1) oder Copolymere. Erfindungsgemäße Copolymere können dabei neben einer oder mehreren Strukturen gemäß Formel (1) potenziell eine oder mehrere weitere Strukturen aus den oben aufgeführten Gruppen 1 bis 7 besitzen. Dabei kann die Konjugationsunterbrechung durch Einheiten gemäß Formel (1) in Nachbarschaft zu jeder der Struktureinheiten der Gruppen 1 bis 7 erfolgen. Die erfindungsgemäßen Copolymere können statistische, alternierende oder blockartige Strukturen aufweisen oder auch mehrere dieser Strukturen abwechselnd besitzen. Wie Copolymere mit blockartigen Strukturen erhalten werden können, ist beispielsweise in WO 05/014688 beschrieben. Ebenso sei an dieser Stelle hervorgehoben, dass das Polymer nicht zwangsläufig linear aufgebaut ist, sondern auch verzweigt sein kann, bzw. auch dendritische Strukturen aufweisen kann.

Die erfindungsgemäßen Polymere weisen im Allgemeinen 10 bis 10000, bevorzugt 20 bis 5000, besonders bevorzugt 50 bis 2000 Wiederholeinheiten auf.

Die erfindungsgemäßen Polymere werden in der Regel durch Polymerisation von einer oder mehreren Monomersorten hergestellt, von denen mindestens ein Monomer im Polymer zu Einheiten gemäß Formel (1) führt. Entsprechende Polymerisationsreaktionen gibt es prinzipiell viele. Es haben sich hier jedoch einige Typen besonders bewährt, die zu C-C- bzw. zu C-N-Verknüpfungen führen:
(A) Polymerisation gemäß Suzuki;
(B) Polymerisation gemäß Yamamoto;
(C) Polymerisation gemäß Stille;
(D) Polymerisation gemäß Hartwig-Buchwald.

Wie die Polymerisation nach diesen Methoden durchgeführt werden kann und wie die Polymere vom Reaktionsmedium abgetrennt und gereinigt werden können, ist beispielsweise in WO 03/048225 und in WO 04/022626 beschrieben. Wie besonders reine Polymere erhalten werden können, ist beispielsweise in EP 04023475.9 beschrieben.

Zur Synthese der Polymere werden die entsprechenden Monomere benötigt. Für die Synthese von Einheiten aus Gruppe 1 bis 7 sei auf die oben genannte Literatur verwiesen.

Monomere, die in erfindungsgemäßen Polymeren zu Struktureinheiten gemäß Formel (1) führen, sind entsprechende Spirobifluoren-Derivate, die an geeigneten Positionen an beiden Molekülhälften, insbesondere in der 4,4'-Position oder in der 1,1'-Position, geeignete Funktionalitäten aufweisen, die es erlauben, diese Monomereinheit in das Polymer einzubauen. Besonders geeignete Funktionalitäten für die Polymerisation sind Halogene, insbesondere Brom, oder Sulfonate oder für die Polymerisation gemäß Suzuki auch Boronsäurederivate oder für die Polymerisation gemäß Stille auch Trialkylzinnderivate. Die Synthese von 4,4'-Dibromspirobifluoren-Derivaten wurde von X. Cheng et al. (Org. Lett. 2004, 6, 2381-2383) beschrieben. Diese Synthese ermöglicht auch die Einführung von Substituenten in der 1,1'-Position. Aus den Brom-Derivaten lassen sich durch Standard-Reaktionen, wie sie dem Fachmann der organischen Synthese geläufig sind, ebenfalls die Boronsäure-Derivate erzeugen.

Ebenso lassen sich aus den oben beschriebenen 4,4'-Dibrom-1,1'-dialkoxy-Derivaten entsprechende Monomere für die Polymerisation über die 1,1'-Positionen erzeugen. Dafür werden zunächst die 4,4'-Positionen durch Umsetzung des Broms blockiert (beispielsweise durch Kupplung mit einem Boronsäurederivat oder durch Reduktion). Die Alkoxygruppen werden in reaktive funktionelle Gruppen umgewandelt, beispielsweise in Triflatgruppen, die entweder direkt in der Polymerisation eingesetzt werden können oder die beispielsweise in einer palladiumkatalysierten Reaktion in ein Boronsäurederivat umgewandelt werden können.

Ein weiterer Gegenstand der Erfindung sind bifunktionelle monomere Verbindungen gemäß Formel (2), 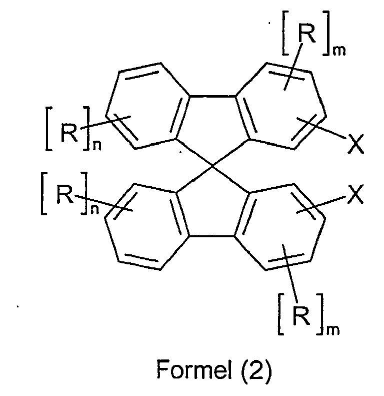 wobei R¹, n und m dieselbe Bedeutung haben, wie oben beschrieben, und weiterhin gilt:
- X: ist bei jedem Auftreten gleich oder verschieden eine Gruppe ausgewählt aus der Gruppe bestehend aus Cl, Br, I, O-Tosylat, O-Triflat, O-SO₂R¹, B(OR¹)₂ und Sn(R¹)₃, vorzugsweise aus Br, O-Triflat und B(OR¹)₂, wobei R¹ dieselbe Bedeutung hat, wie oben beschrieben, und wobei zwei oder mehr Reste R¹ auch miteinander ein Ringsystem bilden können.
- R: ist bei jedem Auftreten gleich oder verschieden CN, NO₂, OH, N(R¹)₂, Si(R¹)₃, B(R¹)₂, eine geradkettige Alkyl-, Alkoxy- oder Thioalkoxygruppe mit 1 bis 40 C- Atomen oder eine verzweigte oder cyclische Alkyl-, Alkoxy- oder Thioalkoxygruppe mit 3 bis 40 C-Atomen, in der jeweils auch ein oder mehrere nicht benachbarte C-Atome durch -CR¹=CR¹-, -C≡C-, -NR¹-, -O-, -S-, -CO-O-, C=O, P(=O)R¹, SO, SO₂ oder -O-CO-O- ersetzt sein können und wobei auch ein oder mehrere H-Atome durch Fluor ersetzt sein können, ein aromatisches oder heteroaromatisches Ringsystem mit 5 bis 40 aromatischen Ringatomen, welches auch durch einen oder mehrere nicht-aromatische Reste R substituiert sein kann; oder eine Kombination aus 2, 3, 4 oder 5 der oben genannten Gruppen; dabei können auch zwei oder mehrere der Reste R miteinander ein aliphatisches oder aromatisches, mono- oder polycyclisches Ringsystem bilden;
wobei die folgende Verbindung von der Erfindung ausgeschlossen ist: 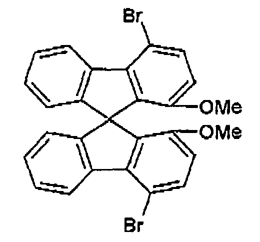 und weiterhin mit der Maßgabe, dass die beiden Gruppen X nicht in 2 und 2'-Position gebunden sind.

Es kann außerdem bevorzugt sein, das erfindungsgemäße Polymer nicht als Reinsubstanz, sondern als Mischung (Blend) zusammen mit weiteren beliebigen polymeren, oligomeren, dendritischen und/oder niedermolekularen Substanzen zu verwenden. Diese können beispielsweise die elektronischen Eigenschaften verbessern oder selber emittieren. So ist beispielsweise eine bevorzugte Ausführungsform der Erfindung das Zumischen einer Verbindung, die bei Raumtemperatur mit hoher Effizienz aus dem Triplett-Zustand Licht emittieren kann, so dass dann die Mischung befähigt ist, mit hoher Effizienz aus dem Triplett-Zustand Licht zu emittieren. Derartige Verbindungen sind oben bereits als weitere Strukturelemente für das Polymer beschrieben. Für die zugemischten Verbindungen gelten dieselben Bevorzugungen, wie oben bereits beschrieben. Aber auch elektronisch inerte Blendbestandteile können sinnvoll sein, um beispielsweise die Viskosität einer Lösung oder die Morphologie des gebildeten Films zu kontrollieren. Solche Blends sind daher auch Bestandteil der vorliegenden Erfindung.

Gegenstand der Erfindung sind weiterhin Lösungen und Formulierungen aus einem oder mehreren erfindungsgemäßen Polymeren oder Blends in einem oder mehreren Lösungsmitteln. Wie Polymerlösungen hergestellt werden können, ist beispielsweise in WO 02/072714, WO 03/019694 und der darin zitierten Literatur beschrieben. Diese Lösungen können verwendet werden, um dünne Polymerschichten herzustellen, zum Beispiel durch Flächenbeschichtungsverfahren (z. B. Spincoating) oder durch Druckverfahren (z. B. InkJet Printing).

Die erfindungsgemäßen Polymere und Blends können in PLEDs verwendet werden. Wie PLEDs hergestellt werden können, wird als allgemeines Verfahren ausführlich in WO 04/037887 beschrieben, das entsprechend für den Einzelfall anzupassen ist. Wie oben beschrieben, eignen sich die erfindungsgemäßen Polymere ganz besonders als Elektrolumineszenzmaterialien in derart hergestellten PLEDs oder Displays.

Als Elektrolumineszenzmaterialien im Sinne der Erfindung gelten Materialien, die als aktive Schicht in einer PLED Verwendung finden können. Aktive Schicht bedeutet, dass die Schicht befähigt ist, bei Anlegen eines elektrischen Feldes Licht abzustrahlen (lichtemittierende Schicht) und/oder dass sie die Injektion und/oder den Transport von positiven und/oder negativen Ladungen verbessert (Ladungsinjektions- oder Ladungstransportschicht).

Gegenstand der Erfindung ist daher auch die Verwendung eines erfindungsgemäßen Polymers oder Blends in einer PLED, insbesondere als Elektrolumineszenzmaterial.

Gegenstand der Erfindung ist ebenfalls eine PLED mit einer oder mehreren aktiven Schichten, wobei mindestens eine dieser aktiven Schichten ein oder mehrere erfindungsgemäße Polymere oder Blends enthält. Die aktive Schicht kann beispielsweise eine lichtemittierende Schicht und/oder eine Transportschicht und/oder eine Ladungsinjektionsschicht sein.

Die erfindungsgemäßen Polymere weisen gegenüber den in WO 03/020790 beschriebenen Poly-Spirobifluorenen, die über die 2,7-Positionen im Polymer verknüpft sind, und gegenüber Poly-Spirobifluorenen gemäß WO 97/20877 und EP 0882082, die über die 2,2'-Positionen verknüpft sind, die hier als nächstliegender Stand der Technik genannt werden, folgende überraschenden Vorteile auf:
(1) Die Lebensdauer unter PM-Ansteuerung ist höher als bei vergleichbaren Polymeren, die bei ähnlicher Zusammensetzung keine Einheiten gemäß Formel (1) enthalten, sondern statt dessen Spirobifluorene, die über die 2,7- oder insbesondere über die 2,2'-Positionen verknüpft sind. Eine Verbesserung der Lebensdauer ist für die Anwendung von enormer Bedeutung, da insbesondere bei blau und grün emittierenden Polymeren die unzureichende Lebensdauer bislang noch das größte Hindernis für eine technische Anwendung ist. Dieses Ergebnis ist überraschend gegenüber konjugierten Polymeren auf Basis von 2,7-verknüpften Spirobifluorenen, da man bislang davon ausgegangen war, dass eine hohe Konjugation eine notwendige Voraussetzung für gute Lebensdauer ist. Die bessere Lebensdauer gegenüber Polymeren, die 2,2'-verknüpfte Spirobifluoren-Einheiten enthalten, ist ebenfalls unerwartet, da auch diese Polymere teilkonjugiert sind und ähnliche Einheiten enthalten.
(2) Die erfindungsgemäßen Polymere weisen, bei ansonsten gleicher Zusammensetzung, höhere Leuchteffizienzen bei hoher Leuchtdichte in der Anwendung auf. Dies ist von enormer Bedeutung, da somit entweder gleiche Helligkeit bei geringerem Energieverbrauch erzielt werden kann, was vor allem bei mobilen Applikationen (Displays für Handys, Pager, PDA, etc.), die auf Batterien und Akkus angewiesen sind, sehr wichtig ist. Umgekehrt erhält man bei gleichem Energieverbrauch höhere Helligkeiten, was beispielsweise für Beleuchtungsanwendungen interessant sein kann.
(3) Insbesondere im Vergleich zu 2,7-verknüpften Spirobifluoren-Polymeren ist eine tiefer blaue Emissionsfarbe mit einer schärferen Emissionsbande erzielbar. Daher sind die erfindungsgemäßen Polymere besser geeignet als Polymere gemäß dem Stand der Technik zur Einpolymerisation eines anderen blauen Emitters, da dadurch die Energie besser auf den Emitter übertragen werden kann.
(4) Gerade im Vergleich zu 2,7-verknüpften Spirobifluoren-Polymeren, die Triplett-Emitter enthalten, lässt sich mit den erfindungsgemäßen Polymeren ein besserer Energieübertrag auf das Polymer erreichen und ein geringerer Energierückübertrag auf das restliche Polymer. Dadurch erhält man einerseits reinere Emissionsfarben und andererseits eine höhere Effizienz.

Im vorliegenden Anmeldetext und auch in den im Weiteren folgenden Beispielen wird auf die Verwendung der erfindungsgemäßen Polymere in Bezug auf PLEDs und entsprechende Displays abgezielt. Trotz dieser Beschränkung der Beschreibung ist es für den Fachmann ohne weiteres erfinderisches Zutun möglich, die erfindungsgemäßen Polymere als Halbleiter (oder bei geeigneter Dotierung auch Leiter) auch für weitere Verwendungen in anderen elektronischen Devices (Vorrichtungen) zu benutzen, z. B. in organischen Feld-Effekt-Transistoren (O-FETs), organischen integrierten Schaltungen (O-ICs), organischen Dünnfilmtransistoren (O-TFTs), organischen lichtemittierenden Transistoren (O-LETs), lichtemittierenden elektrochemischen Zellen (LECs), organischen Solarzellen (O-SCs), organischen Feld-Quench-Devices (O-FQD, z. B. gemäß M. Redecker et al., Proc. 22nd International Display Research Conf., Nice 2002, S. 97-99) oder auch organischen Laserdioden (O-Laser). Die Verwendung der erfindungsgemäßen Polymere in den entsprechenden Vorrichtungen ist ebenfalls ein Gegenstand der vorliegenden Erfindung. Weiterhin Gegenstand der Erfindung sind organische Feld-Effekt-Transistoren (O-FETs), organische integrierte Schaltungen (O-ICs), organische Dünnfilmtransistoren (O-TFTs), organische Solarzellen (O-SCs), organische Feld-Quench-Devices (O-FQDs), organische lichtemittierende Transistoren (O-LETs), lichtemitterende elektrochemische Zellen (LECs) und organische Laserdioden (O-Laser), enthaltend mindestens ein erfindungsgemäßes Polymer.

Ebenso ist es für den Fachmann ein Leichtes, die oben gemachten Beschreibungen für Polymere ohne weiteres erfinderisches Zutun auf entsprechende Dendrimere oder Oligomere zu übertragen. Derartige Dendrimere und Oligomere sind ebenfalls ein Gegenstand der vorliegenden Erfindung.

### Beispiele:

### Beispiel 1: Synthese von Monomeren, die in Polymeren zu Einheiten gemäß Formel (1) führen

Die Synthese von 4,4'-Dibrom-1,1'-dimethoxy-spirobifluoren (Monomer **M1**) erfolgte gemäß der Literatur (X. Cheng et al., Org. Lett. 2004, 6, 2381-2383).

### Beispiel 2: Synthese weiterer Monomere

Die Synthese der Monomere **M2** bis **M5** ist in WO 03/020790 und der darin zitierten Literatur beschrieben. Die Synthese von 2,2'-Dibromspirobifluoren (Monomer **M6**), das für das Vergleichspolymer verwendet wurde, wurde gemäß F.-I. Wu et al. (J. Mater. Chem. 2002, 12, 2893-2897) durchgeführt. Die Strukturen der weiteren Monomere für erfindungsgemäße Polymere und Vergleichspolymere sind im Folgenden abgebildet.

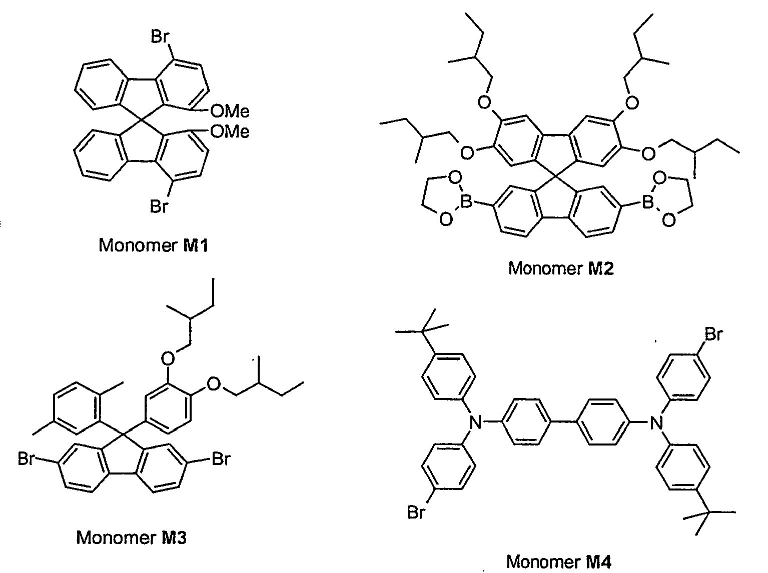

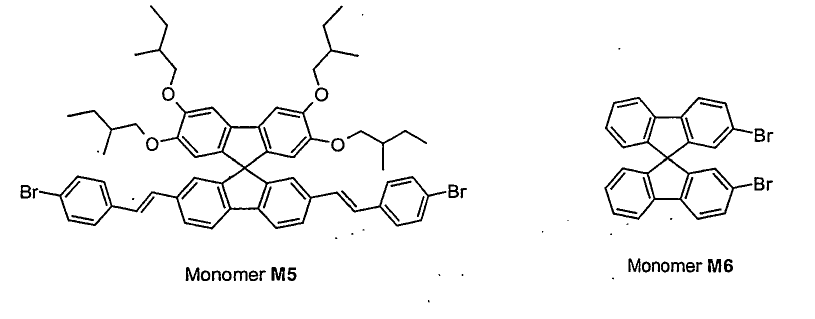

### Beispiel 3: Synthese der Polymere

Die Polymere wurden durch SUZUKI-Kupplung gemäß WO 03/048225 synthetisiert. Die Zusammensetzung der synthetisierten Polymere **P1** bis **P3** ist in Tabelle 1 zusammengefasst. Außerdem wurden die Vergleichspolymere **V1** und **V2** synthetisiert, die statt des Monomers **M1,** das im Polymer zu Einheiten gemäß Formel (1) führt, das Monomer **M3** bzw. das Monomer **M6** enthalten. Die Zusammensetzung dieser Vergleichspolymere ist ebenfalls in Tabelle 1 aufgeführt.

### Beispiel 4: Herstellung der PLEDs

Die Polymere wurden für einen Einsatz in PLEDs untersucht. Die PLEDs waren jeweils Zweischichtsysteme, d. h. Substrat//ITO//PEDOT//Polymer//Kathode. PEDOT ist ein Polythiophen-Derivat (Baytron P, von H. C. Starck, Goslar). Als Kathode wurde in allen Fällen Ba/Ag (Aldrich) verwendet. Wie PLEDs dargestellt werden können, ist in WO 04/037887 und der darin zitierten Literatur ausführlich beschrieben.

### Beispiel 5 bis 10: Device-Beispiele

Die Ergebnisse, die bei Verwendung der Polymere **P1** bis **P3** in PLEDs erhalten wurden, sind in Tabelle 1 zusammengefasst. Ebenso aufgeführt sind die Elektrolumineszenz-Ergebnisse, die unter Verwendung der Vergleichspolymere **V1** bis **V3** erhalten wurden.

Wie man aus den Ergebnissen erkennen kann, ist die Effizienz der Vergleichspolymere und der erfindungsgemäßen Polymere bei niedriger Effizienz vergleichbar. Bei höherer Effizienz ist jedoch die Effizienz der erfindungsgemäßen Polymere deutlich höher als die der Vergleichspolymere. Dies zeigt, dass die erfindungsgemäßen Polymere besser für den Einsatz in Displays mit PM-Ansteuerung geeignet sind als Polymere gemäß dem Stand der Technik. Zudem ist die Lebensdauer der erfindungsgemäßen Polymere unter PM-Ansteuerung höher als die der Polymere gemäß dem Stand der Technik unter gleichen Bedingungen.

**Tabelle 1: Device-Ergebnisse mit erfindungsgemäßen Polymeren und Vergleichpolymeren**

| Beispiel | Polymer | Monomer für Einheiten gemäß Formel (1) | Weitere Monomere | Max. Eff. / cd/A | Eff. @ 2000 cd/m² / cd/A | U @ 100 cd/m² / V | CIE x/y^{a} | Lebensdauer^{b} / h |
|---|---|---|---|---|---|---|---|---|
| 5 | **P1** | 10% **M1** | 50% **M2**, 20% **M3**, 10% **M4**, 10% **M5** | 4.54 | 3.9 | 3.9 | 0.17 / 0.30 | 450 |
| 6 | **P2** | 30% **M1** | 50% **M2**, 10% **M4**, 10% **M5** | 4.08 | | 4.9 | 0.16 / 0.25 | 300 |
| 7 | **P3** | 10% **M1** | 50% **M2**, 30% **M4**, 10% **M5** | 2.40 | | 3.8 | 0.16/0.24 | |
| 8 (Vergleich) | **V1** | --- | 50% **M2**, 30% **M3,** 10% **M4**, 10% **M5** | 4.66 | 3.4 | 3.9 | 0.17 / 0.30 | 350 |
| 9 (Vergleich) | **V2** | --- | 50% **M2**, 20% **M3,** 10% **M4,** 10% **M5,** 10% **M6** | 4.10 | | 4.0 | 0.17 / 0.29 | 340 |
| 10 (Vergleich) | **V3** | --- | 50% **M2**, 10% **M3**, 30% **M4**, 10% **M5** | 2.29 | | 3.9 | 0.17 / 0.23 | |

| | | | | | | | | |
|---|---|---|---|---|---|---|---|---|
| ^{a} CIE-Koordinaten: Farbkoordinaten der Commision Internationale de l'Eclairage 1931. ^{b} Lebensdauer: Zeit bis zum Abfall der Helligkeit auf 50 % der Anfangshelligkeit, MUX64 (100 Hz, Pulsdauer 156.25 µs, mittlere Anfangshelligkeit 400 cd/m², maximale Spitzenhelligkeit, 25600 cd/m²) | | | | | | | | |

## Patentansprüche

1. Polymere, enthaltend mindestens 1 mol% einer ersten Wiederholeinheit gemäß
Formel (1), 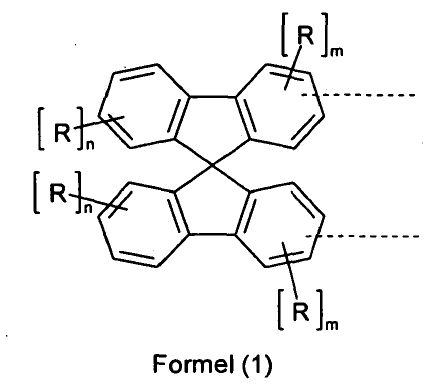 wobei die verwendeten Symbole und Indizes die folgende Bedeutung besitzen:
R ist bei jedem Auftreten gleich oder verschieden F, Cl, Br, I, CN, NO₂, OH, N(R¹)₂, Si(R¹)₃, B(R¹)₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, in der jeweils auch ein oder mehrere nicht benachbarte C-Atome durch -CR¹=CR¹, -C≡C-, -NR¹-, -O-, -S-, -CO-O-, C=O, P(=O)R¹, SO, SO₂ oder -O-CO-O- ersetzt sein können und wobei auch ein oder mehrere H-Atome durch Fluor ersetzt sein können, oder ein aromatisches oder heteroaromatisches Ringsystem mit 5 bis 40 aromatischen C-Atomen, welches auch durch einen oder mehrere nicht-aromatische Reste R substituiert sein kann; oder eine Kombination aus 2, 3, 4 oder 5 der oben genannten Gruppen; dabei können auch zwei oder mehrere der Reste R miteinander ein aliphatisches oder aromatisches, mono- oder polycyclisches Ringsystem bilden;
R¹ ist bei jedem Auftreten gleich oder verschieden H oder ein aliphatischer oder aromatischer Kohlenwasserstoffrest mit 1 bis 20 C-Atomen;
n ist bei jedem Auftreten gleich oder verschieden 0, 1, 2, 3 oder 4;
m ist bei jedem Auftreten gleich oder verschieden 0, 1, 2 oder 3;
die gestrichelten Bindungen deuten dabei die Verknüpfung im Polymer an;
mit der Maßgabe, dass die Verknüpfung nicht über die 2,2'-Positionen erfolgt; und enthaltend mindestens 1 mol% einer zweiten Wiederholeinheit, die entweder gleich einer Wiederholeinheit gemäß Formel (1) ist oder verschieden ist.

2. Polymere gemäß Anspruch 1, **dadurch gekennzeichnet, dass** für die Symbole und Indizes gilt:
R ist bei jedem Auftreten gleich oder verschieden F, CN, NO₂, OH, N(R¹)₂, eine geradkettige Alkyl-, Alkoxy- oder Thioalkoxygruppe mit 1 bis 20 C- Atomen oder eine verzweigte oder cyclische Alkyl-, Alkoxy- oder Thioalkoxygruppe mit 3 bis 20 C-Atomen, in der jeweils auch ein oder mehrere nicht benachbarte C-Atome durch -CR¹=CR¹, -C≡C-, -NR¹-, -O-, -S-, C=O oder P(=O)R¹ ersetzt sein können und wobei auch ein oder mehrere H-Atome durch Fluor ersetzt sein können, oder ein aromatisches oder heteroaromatisches Ringsystem mit 5 bis 30 aromatischen Ringatomen, welches auch durch einen oder mehrere nicht-aromatische Reste R substituiert sein kann; oder eine Kombination aus 2, 3 oder 4 der oben genannten Gruppen; dabei können auch zwei oder mehrere der Reste R miteinander ein aliphatisches oder aromatisches, mono- oder polycyclisches Ringsystem bilden;
R¹ ist wie in Anspruch 1 beschrieben;
n ist bei jedem Auftreten gleich oder verschieden 0, 1, 2 oder 3;
m ist bei jedem Auftreten gleich oder verschieden 0, 1 oder 2.

3. Polymere gemäß Anspruch 1 und/oder 2, **dadurch gekennzeichnet, dass** die Einheiten gemäß Formel (1) an den beiden Spiro-Molekülhälften symmetrisch substituiert sind.

4. Polymere gemäß einem oder mehreren der Ansprüche 1 bis 3, **dadurch gekennzeichnet, dass** die Verknüpfung der Einheiten gemäß Formel (1) über gleiche Positionen an den beiden Spiro-Molekülhälften erfolgt.

5. Polymere gemäß Anspruch 4, **dadurch gekennzeichnet, dass** die Verknüpfung der Einheiten gemäß Formel (1) über die 1,1'-Positionen oder über die 4,4'-Positionen erfolgt.

6. Polymere gemäß einem oder mehreren der Ansprüche 1 bis 5, **dadurch gekennzeichnet, dass** sie teilkonjugiert sind.

7. Polymere gemäß einem oder mehreren der Ansprüche 1 bis 6, **dadurch gekennzeichnet, dass** als weitere Einheiten aromatische, carbocyclische Strukturen mit 6 bis 40 C-Atomen anwesend sind, die substituiert oder unsubstituiert sein können, wobei als Substituenten die in Anspruch 1 definierten Reste R in Frage kommen.

8. Polymere gemäß Anspruch 7, **dadurch gekennzeichnet, dass** die weiteren Einheiten ausgewählt sind aus der Gruppe der Fluoren-Derivate, der 2,7-Spirobifluoren-Derivate, der Dihydrophenanthren-Derivate, der cis- oder trans-Indenofluoren-Derivate, der 1,4-Phenylen-Derivate, der 4,4'-Biphenylylen-Derivate, der 4,4"-Terphenylylen-Derivate, der 2,7- oder 3,6-Phenanthren-Derivate, der Dihydropyren- und/oder Tetrahydropyren-Derivate.

9. Polymere gemäß einem oder mehreren der Ansprüche 1 bis 8, **dadurch gekennzeichnet, dass** weitere Strukturelemente anwesend sind, die die Morphologie und gegebenenfalls auch die Emissionsfarbe der resultierenden Polymere beeinflussen, ausgewählt aus der Gruppe der durch R substituierten oder unsubstituierten kondensierten aromatischen Strukturen mit 6 bis 40 C-Atomen oder Tolan-, Stilben- oder Bisstyrylarylenderivate.

10. Polymere gemäß einem oder mehreren der Ansprüche 1 bis 9, **dadurch gekennzeichnet, dass** weitere Strukturelemente anwesend sind, die die Lochinjektions- und/oder Lochtransporteigenschaften der Polymere erhöhen, ausgewählt aus der Gruppe der aromatischen Amine oder Phosphine oder elektronenreichen Heterocyclen.

11. Polymere gemäß einem oder mehreren der Ansprüche 1 bis 10, **dadurch gekennzeichnet, dass** weitere Strukturelemente anwesend sind, die die Elektroneninjektions- und/oder Elektronentransporteigenschaften der Polymere erhöhen, ausgewählt aus der Gruppe der elektronenarmen Aromaten oder Heterocyclen oder der Triarylborane.

12. Polymere gemäß einem oder mehreren der Ansprüche 1 bis 11, **dadurch gekennzeichnet, dass** weitere Strukturelemente Einheiten gemäß Anspruch 10 und Einheiten gemäß Anspruch 11 aneinander gebunden enthalten.

13. Polymere gemäß einem oder mehreren der Ansprüche 1 bis 12, **dadurch gekennzeichnet, dass** weitere Strukturelemente anwesend sind, welche auch bei Raumtemperatur mit hoher Effizienz aus dem Triplettzustand Licht emittieren können.

14. Polymere gemäß Anspruch 13, **dadurch gekennzeichnet, dass** diese Strukturelemente Elemente der Gruppen 8 bis 10 (Ru, Os, Rh, Ir, Pd, Pt) enthalten.

15. Polymere gemäß Anspruch 13 und/oder 14, **dadurch gekennzeichnet, dass** weitere Strukturelemente anwesend sind, welche den Übergang vom Singulettzum Triplett-Zustand verbessern, ausgewählt aus der Gruppe der Carbazol-Derivate, der Keto-, Phosphinoxid-, Sulfoxid-, Sulfon- oder Silaneinheiten.

16. Polymere gemäß einem oder mehreren der Ansprüche 1 bis 15, **dadurch gekennzeichnet, dass** sie neben Einheiten gemäß Formel (1) mindestens 30 mol% Einheiten gemäß Anspruch 7 und/oder 8 enthalten.

17. Polymere gemäß einem oder mehreren der Ansprüche 1 bis 16, **dadurch gekennzeichnet, dass** sie neben Einheiten gemäß Formel (1) mindestens 5 mol% Einheiten gemäß Anspruch 10 und/oder 11, die die Ladungstransporteigenschaften verbessern, enthalten.

18. Polymere gemäß einem oder mehreren der Ansprüche 1 bis 17, **dadurch gekennzeichnet, dass** der Anteil an Einheiten gemäß Formel (1) zwischen 5 und 70 mol% liegt.

19. Polymere gemäß einem oder mehreren der Ansprüche 1 bis 18, **dadurch gekennzeichnet, dass** die Polymere statistische, alternierende oder blockartige Strukturen aufweisen oder auch mehrere dieser Strukturen abwechselnd besitzen und linear, verzweigt oder dendritisch aufgebaut sind.

20. Mischungen (Blends) enthaltend ein oder mehrere Polymere gemäß einem oder mehreren der Ansprüche 1 bis 19 zusammen mit weiteren polymeren, oligomeren, dendritischen und/oder niedermolekularen Substanzen.

21. Mischungen gemäß Anspruch 20, **dadurch gekennzeichnet, dass** die zugemischte Verbindung eine Verbindung ist, die bei Raumtemperatur aus dem Triplett-Zustand Licht emittieren kann.

22. Lösungen und Formulierungen aus einem oder mehreren Polymeren oder Blends gemäß einem oder mehreren der Ansprüche 1 bis 21 in einem oder mehreren Lösungsmitteln.

23. Bifunktionelle monomere Verbindungen gemäß Formel (2), 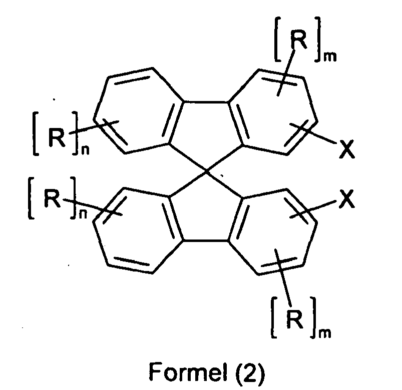 wobei R¹, n und m dieselbe Bedeutung haben, wie in Anspruch 1 beschrieben, und weiterhin gilt:
X ist bei jedem Auftreten gleich oder verschieden eine Gruppe ausgewählt aus der Gruppe bestehend aus Cl, Br, I, O-Tosylat, O-Triflat, O-SO₂R¹, B(OR¹)₂ und Sn(R¹)₃, wobei R¹ dieselbe Bedeutung hat, wie in Anspruch 1 beschrieben, und wobei zwei oder mehr Reste R¹ auch miteinander ein Ringsystem bilden können,
R ist bei jedem Auftreten gleich oder verschieden CN, NO₂, OH, N(R¹)₂, Si(R¹)₃, B(R¹)₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, in der jeweils auch ein oder mehrere nicht benachbarte C-Atome durch -CR¹=CR¹-, -C≡C-, -NR¹-, -O-, -S-, -CO-O-, C=O, P(=O)R¹, SO, SO₂ oder -O-CO-O- ersetzt sein können und wobei auch ein oder mehrere H-Atome durch Fluor ersetzt sein können, ein aromatisches oder heteroaromatisches Ringsystem mit 5 bis 40 aromatischen Ringatomen, welches auch durch einen oder mehrere nicht-aromatische Reste R substituiert sein kann; oder eine Kombination aus 2, 3, 4 oder 5 der oben genannten Gruppen; dabei können auch zwei oder mehrere der Reste R miteinander ein aliphatisches oder aromatisches, mono- oder polycyclisches Ringsystem bilden;
wobei die folgende Verbindung von der Erfindung ausgeschlossen ist: 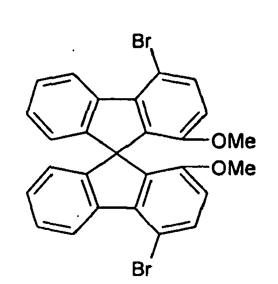 und weiterhin mit der Maßgabe, dass die beiden Gruppen X nicht in 2 und 2'-Position gebunden sind.

24. Verwendung eines Polymers oder Blends gemäß einem oder mehreren der Ansprüche 1 bis 21 in einer organischen elektronischen Vorrichtung, insbesondere als Elektrolumineszenzmaterial.

25. Organische elektronische Vorrichtung mit einer oder mehreren aktiven Schichten, wobei mindestens eine dieser aktiven Schichten ein oder mehrere Polymere oder Blends gemäß einem oder mehreren der Ansprüche 1 bis 21 enthält.

26. Organische elektronische Vorrichtung gemäß Anspruch 25, **dadurch gekennzeichnet, dass** es sich um eine polymere Leuchtdiode (PLED), einen organischen Feld-Effekt-Transistor (O-FET), eine organische integrierte Schaltung (O-IC), einen organischen Dünnfilmtransistor (O-TFT), eine organische Solarzelle (O-SC), ein organisches Feld-Quench-Device (O-FQD), einen organischen lichtemittierenden Transistor (O-LET), eine lichtemittierende elektrochemische Zelle (LEC) oder eine organische Laserdiode (O-Laser) handelt.

## Claims

1. Polymers comprising at least 1 mol% of a first recurring unit of the formula (1), 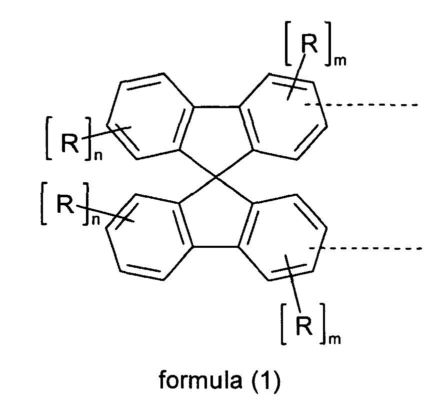 where the symbols and indices used have the following meanings:
R is on each occurrence, identically or differently, F, Cl, Br, I, CN, NO₂, OH, N(R¹)₂, Si(R¹)₃, B(R¹)₂, a straight-chain alkyl, alkoxy or thioalkoxy group having 1 to 40 C atoms or a branched or cyclic alkyl, alkoxy or thioalkoxy group having 3 to 40 C atoms, in which, in addition, one or more non- adjacent C atoms may each be replaced by -CR¹=CR¹, -C≡C-, -NR¹-, -O-, -S-, -CO-O-, C=O, P(=O)R¹, SO, SO₂ or -O-CO-O- and where, in addition, one or more H atoms may be replaced by fluorine, or an aromatic or het- eroaromatic ring system having 5 to 40 aromatic C atoms, which may also be substituted by one or more non-aromatic radicals R; or a combination of 2, 3, 4 or 5 of the above-mentioned groups; in addition, two or more of the radicals R here may form an aliphatic or aromatic, mono- or polycyclic ring system with one another;
R¹ is on each occurrence, identically or differently, H or an aliphatic or aro- matic hydrocarbon radical having 1 to 20 C atoms;
n is on each occurrence, identically or differently, 0, 1, 2, 3 or 4;
m is on each occurrence, identically or differently, 0, 1, 2 or 3;
the dashed bonds here indicate the linking in the polymer;
with the proviso that the linking does not take place via the 2,2'-positions;
and comprising at least 1 mol% of a second recurring unit which is either identical to or different from a recurring unit of the formula (1).

2. Polymers according to Claim 1, **characterised in that** the following applies to the symbols and indices:
R is on each occurrence, identically or differently, F, CN, NO₂, OH, N(R¹)₂, a straight-chain alkyl, alkoxy or thioalkoxy group having 1 to 20 C atoms or a branched or cyclic alkyl, alkoxy or thioalkoxy group having 3 to 20 C atoms, in which, in addition, one or more non-adjacent C atoms may each be replaced by -CR¹=CR¹-, -C≡C-, -NR¹-, -O-, -S-, C=O or P(=O)R¹ and where, in addition, one or more H atoms may be replaced by fluorine, or an aro- matic or heteroaromatic ring system having 5 to 30 aromatic ring atoms, which may also be substituted by one or more non-aromatic radicals R; or a combination of 2, 3 or 4 of the above-mentioned groups; in addition, two or more of the radicals R here may form an aliphatic or aromatic, mono- or polycyclic ring system with one another;
R¹ is as described in Claim 1;
n is on each occurrence, identically or differently, 0, 1, 2 or 3;
m is on each occurrence, identically or differently, 0, 1 or 2.

3. Polymers according to Claim 1 and/or 2, **characterised in that** the units of the formula (1) are symmetrically substituted on the two spiro moieties.

4. Polymers according to one or more of Claims 1 to 3, **characterised in that** the units of the formula (1) are linked via identical positions on the two spiro moieties.

5. Polymers according to Claim 4, **characterised in that** the units of the formula (1) are linked via the 1,1'-positions or via the 4,4'-positions.

6. Polymers according to one or more of Claims 1 to 5, **characterised in that** they are partially conjugated.

7. Polymers according to one or more of Claims 1 to 6, **characterised in that** further units present are aromatic, carbocyclic structures having 6 to 40 C atoms, which may be substituted or unsubstituted, where suitable substituents are the radicals R defined in Claim 1.

8. Polymers according to Claim 7, **characterised in that** the further units are selected from the group of the fluorene derivatives, 2,7-spirobifluorene derivatives, dihydrophenanthrene derivatives, cis- or trans-indenofluorene derivatives, 1,4-phenylene derivatives, 4,4'-biphenylylene derivatives, 4,4"-terphenylylene derivatives, 2,7- or 3,6-phenanthrene derivatives, dihydropyrene derivatives and/or tetrahydropyrene derivatives.

9. Polymers according to one or more of Claims 1 to 8, **characterised in that** further structural elements are present which influence the morphology and optionally also the emission colour of the resultant polymers, selected from the group of the R-substituted or unsubstituted condensed aromatic structures having 6 to 40 C atoms or tolan, stilbene or bisstyrylarylene derivatives.

10. Polymers according to one or more of Claims 1 to 9, **characterised in that** further structural elements are present which improve the hole-injection and/or hole-transport properties of the polymers, selected from the group of the aromatic amines or phosphines or electron-rich heterocycles.

11. Polymers according to one or more of Claims 1 to 10, **characterised in that** further structural elements are present which improve the electron-injection and/or electron-transport properties of the polymers, selected from the group of the electron-deficient aromatics or heterocycles or triarylboranes.

12. Polymers according to one or more of Claims 1 to 11, **characterised in that** further structural elements comprise units according to Claim 10 and units according to Claim 11 bonded to one another.

13. Polymers according to one or more of Claims 1 to 12, **characterised in that** further structural elements are present which are able to emit light from the triplet state with high efficiency, even at room temperature.

14. Polymers according to Claim 13, **characterised in that** these structural elements contain elements from groups 8 to 10 (Ru, Os, Rh, Ir, Pd, Pt).

15. Polymers according to Claim 13 and/or 14, **characterised in that** further structural elements are present which improve the transfer from the singlet state to the triplet state, selected from the group of the carbazole derivatives, keto units, phosphine oxide units, sulfoxide units, sulfone units or silane units.

16. Polymers according to one or more of Claims 1 to 15, **characterised in that**, besides units of the formula (1), they comprise at least 30 mol% of units according to Claim 7 and/or 8.

17. Polymers according to one or more of Claims 1 to 16, **characterised in that**, besides units of the formula (1), they comprise at least 5 mol% of units according to Claim 10 and/or 11 which improve the charge-transport properties.

18. Polymers according to one or more of Claims 1 to 17, **characterised in that** the proportion of units of the formula (1) is between 5 and 70 mol%.

19. Polymers according to one or more of Claims 1 to 18, **characterised in that** the polymers have random, alternating or block-like structures or alternatively have a plurality of these structures in an alternating sequence and have a linear, branched or dendritic structure.

20. Mixtures (blends) comprising one or more polymers according to one or more of Claims 1 to 19 together with further polymeric, oligomeric, dendritic and/or low-molecular-weight substances.

21. Mixtures according to Claim 20, **characterised in that** the admixed compound is a compound which is able to emit light from the triplet state at room temperature.

22. Solutions and formulations comprising one or more polymers or blends according to one or more of Claims 1 to 21 in one or more solvents.

23. Bifunctional monomeric compounds of the formula (2) 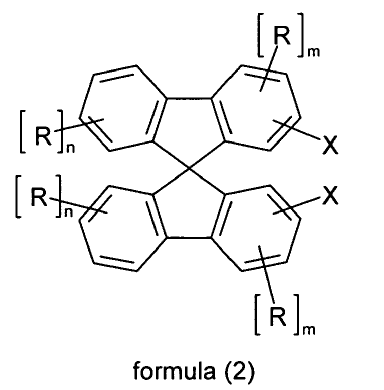 where R¹, n and m have the same meaning as described in Claim 1, and furthermore:
X is on each occurrence, identically or differently, a group selected from the group consisting of Cl, Br, I, O-tosylate, O-triflate, O-SO₂R¹, B(OR¹)₂ and Sn(R¹)₃, where R¹ has the same meaning as described in Claim 1, and where, in addition, two or more radicals R¹ may form a ring system with one another;
R is on each occurrence, identically or differently, CN, NO₂, OH, N(R¹)₂, Si(R¹)₃, B(R¹)₂, a straight-chain alkyl, alkoxy or thioalkoxy group having 1 to 40 C atoms or a branched or cyclic alkyl, alkoxy or thioalkoxy group having 3 to 40 C atoms, in which, in addition, one or more non-adjacent C atoms may each be replaced by -CR¹=CR¹-, -C≡C-, -NR¹-, -O-, -S-, -CO-O-, C=O, P(=O)R¹, SO, SO₂ or -O-CO-O- and where, in addition, one or more H atoms may be replaced by fluorine, an aromatic or heteroaromatic ring system having 5 to 40 aromatic ring atoms, which may also be substituted by one or more non-aromatic radicals R; or a combination of 2, 3, 4 or 5 of the above-mentioned groups; in addition, two or more of the radicals R here may form an aliphatic or aromatic, mono- or polycyclic ring system with one another;
where the following compound is excluded from the invention: 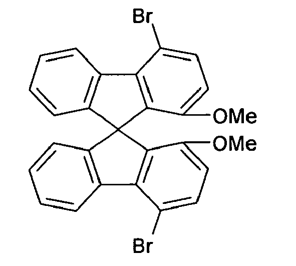 and furthermore with the proviso that the two groups X are not bonded in the 2- and 2'-positions.

24. Use of a polymer or blend according to one or more of Claims 1 to 21 in an organic electronic device, in particular as electroluminescent material.

25. Organic electronic device having one or more active layers, where at least one of these active layers comprises one or more polymers or blends according to one or more of Claims 1 to 21.

26. Organic electronic device according to Claim 25, **characterised in that** it is a polymeric light-emitting diode (PLED), an organic field-effect transistor (O-FET), an organic integrated circuit (O-IC), an organic thin-film transistor (O-TFT), an organic solar cell (O-SC), an organic field-quench device (O-FQD), an organic light-emitting transistor (O-LET), a light-emitting electrochemical cell (LEC) or an organic laser diode (O-laser).

## Revendications

1. Polymères comprenant au moins 1 mol% d'une première unité récurrente de la
formule (1), 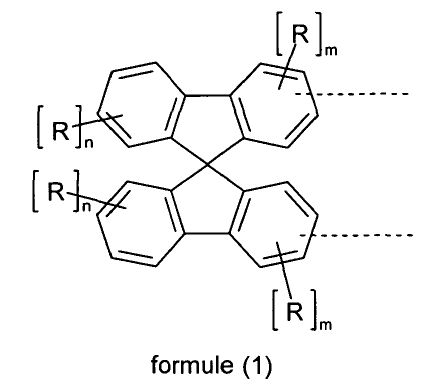 où les symboles et indices utilisés présentent les significations qui suivent :
R est, pour chaque occurrence, de manière identique ou différente, F, CI, Br, I, CN, NO₂, OH, N(R¹)₂, Si(R¹)₃, B(R¹)₂, un groupe alkyle, alcoxy ou thio- alcoxy en chaîne droite comportant de 1 à 40 atomes de C ou un groupe alkyle, alcoxy ou thioalcoxy ramifié ou cyclique comportant de 3 à 40 atomes de C, où, en plus, un ou plusieurs atomes de C non adjacents peu- vent chacun être remplacés par -CR¹=CR¹-, -C≡C-, -NR¹-, -O-, -S-, -CO-O-, C=O, P(=O)R¹, SO, SO₂ ou -O-CO-O- et où, en plus, un ou plusieurs atomes de H peuvent être remplacés par fluor, ou un système de cycle aromatique ou hétéroaromatique comportant de 5 à 40 atomes de C aro- matiques, lequel peut également être substitué par un ou plusieurs radi- caux R non aromatiques ; ou une combinaison de 2, 3, 4 ou 5 des groupes mentionnés ci avant ; en outre, deux des radicaux R afférents ou plus peu- vent former un système de cycle aliphatique ou aromatique, mono- ou polycyclique les uns avec les autres ;
R¹ est, pour chaque occurrence, de manière identique ou différente, H ou un radical hydrocarbone aliphatique ou aromatique comportant de 1 à 20 atomes de C ;
n est, pour chaque occurrence, de manière identique ou différente, 0, 1, 2, 3 ou 4 ;
m est, pour chaque occurrence, de manière identique ou différente, 0, 1, 2 ou 3 ;
les liaisons en pointillés indiquent ici la liaison dans le polymère ; avec la réserve que la liaison n'est pas présente via les positions 2,2' et comprenant au moins 1 mol% d'une seconde unité récurrente qui soit est identique à une unité récurrente de la formule (1), soit en est différente.

2. Polymères selon la revendication 1, **caractérisés en ce que** ce qui suit s'applique aux symboles et indices :
R est, pour chaque occurrence, de manière identique ou différente, F, CN, N₂, OH, N(R¹)₂, un groupe alkyle, alcoxy ou thioalcoxy en chaîne droite comportant de 1 à 20 atomes de C ou un groupe alkyle, alcoxy ou thio- alcoxy ramifié ou cyclique comportant de 3 à 20 atomes de C, où, en plus, un ou plusieurs atomes de C non adjacents peuvent chacun être remplacés par -CR¹=CR¹, -C≡C-, -NR¹-, -O-, -S-, C=O ou P(=O)R¹ et où, en plus, un ou plusieurs atomes de H peuvent être remplacés par fluor, ou un système de cycle aromatique ou hétéroaromatique comportant de 5 à 30 atomes de cycle aromatique, lequel peut également être substitué par un ou plusieurs radicaux R non aromatiques ; ou une combinaison de 2, 3 ou 4 des groupe mentionnés ci avant ; en outre, deux des radicaux R afférents ou plus peu- vent former un système de cycle aliphatique ou aromatiques, mono- ou polycyclique les uns avec les autres ;
R¹ est comme décrit selon la revendication 1 ;
n est, pour chaque occurrence, de manière identique ou différente, 0, 1, 2 ou 3;
m est, pour chaque occurrence, de manière identique ou différente, 0, 1 ou 2.

3. Polymères selon la revendication 1 et/ou 2, **caractérisés en ce que** les unités de la formule (1) sont substituées de façon symétrique sur les deux moitiés spiro.

4. Polymères selon une ou plusieurs des revendications 1 à 3, **caractérisés en ce que** les unités de la formule (1) sont liées via des positions identiques sur les deux moitiés spiro.

5. Polymères selon la revendication 4, **caractérisés en ce que** les unités de la formule (1) sont liées via les positions 1,1' ou via les positions 4,4'.

6. Polymères selon une ou plusieurs des revendications 1 à 5, **caractérisés en ce qu'**ils sont partiellement conjugués.

7. Polymères selon une ou plusieurs des revendications 1 à 6, **caractérisés en ce que** d'autres unités présentes sont des structures aromatiques, carbocycliques comportant de 6 à 40 atomes de C, lesquelles peuvent être substituées ou non substituées, où des substituants appropriés sont les radicaux R définis dans la revendication 1.

8. Polymères selon la revendication 7, **caractérisés en ce que** les autres unités sont choisies parmi le groupe constitué par les dérivés de fluorène, les dérivés de 2,7-spirobifluorène, les dérivés de dihydrophénanthrène, les dérivés de cis- ou trans-indénofluorène, les dérivés de 1,4-phénylène, les dérivés de 4,4'-biphénylylène, les dérivés de 4,4"-terphénylylène, les dérivés de 2,7- ou de 3,6-phénanthrène, les dérivés de dihydropyrène et/ou les dérivés de tétrahydropyrène.

9. Polymères selon une ou plusieurs des revendications 1 à 8, **caractérisés en ce que** d'autres éléments structurels sont présents, lesquels influencent la morphologie et en option, également l'émission de couleurs des polymères résultants, lesquels sont choisis parmi le groupe constitué par les structures aromatiques condensées R-substituées ou non substituées comportant de 6 à 40 atomes de C ou des dérivés de tolane, de stilbène ou de bisstyrylarylène.

10. Polymères selon une ou plusieurs des revendications 1 à 9, **caractérisés en ce que** d'autres éléments structurels sont présents, lesquels améliorent les propriétés d'injection de trous et/ou de transport de trous des polymères, lesquels sont choisis parmi le groupe constitué par les amines ou phosphines aromatiques ou les hétérocycles riches en électrons.

11. Polymères selon une ou plusieurs des revendications 1 à 10, **caractérisés en ce que** d'autres éléments structurels sont présents, lesquels améliorent les propriétés d'injection d'électrons et/ou de transport d'électrons des polymères, lesquels sont choisis parmi le groupe constitué par les aromatiques ou les hétérocycles ou les triarylboranes déficients en électrons.

12. Polymères selon une ou plusieurs des revendications 1 à 11, **caractérisés en ce que** d'autres éléments structurels comprennent des unités selon la revendication 10 et des unités selon la revendication 11 liées les unes aux autres.

13. Polymères selon une ou plusieurs des revendications 1 à 12, **caractérisés en ce que** d'autres éléments structurels sont présents, lesquels peuvent émettre de la lumière depuis l'état de triplet selon une efficacité élevée, même à température ambiante.

14. Polymères selon la revendication 13, **caractérisés en ce que** ces éléments structurels contiennent des éléments pris parmi les groupes 8 à 10 (Ru, Os, Rh, Ir, Pd, Pt).

15. Polymères selon la revendication 13 et/ou 14, **caractérisés en ce que** d'autres éléments structurels sont présents, lesquels améliorent le transfert de l'état de singlet à l'état de triplet, lesquels sont choisis parmi le groupe constitué par les dérivés de carbazole, les unités céto, les unités oxyde de phosphine, les unités sulfoxyde, les unités sulfone ou les unités silane.

16. Polymères selon une ou plusieurs des revendications 1 à 15, **caractérisés en ce que**, en plus des unités de la formule (1), ils comprennent au moins 30 mol% d'unités selon les revendications 7 et/ou 8.

17. Polymères selon une ou plusieurs des revendications 1 à 16, **caractérisés en ce que**, en plus des unités de la formule (1), ils comprennent au moins 5 mol% d'unités selon les revendications 10 et/ou 11, lesquelles améliorent les propriétés de transport de charges.

18. Polymères selon une ou plusieurs des revendications 1 à 17, **caractérisés en ce que** la proportion d'unités de la formule (1) est entre 5 et 70 mol%.

19. Polymères selon une ou plusieurs des revendications 1 à 18, **caractérisés en ce que** les polymères présentent des structures aléatoires, alternées ou similaires à des blocs ou à titre d'alternative, ils comportent une pluralité de ces structures selon une séquence alternée et ils présentent une structure linéaire, ramifiée ou dendritique.

20. Mélanges (blends) comprenant un ou plusieurs polymères selon une ou plusieurs des revendications 1 à 19 en association avec d'autres substances polymériques, oligomériques, dendritiques et/ou de poids moléculaire faible.

21. Mélanges selon la revendication 20, **caractérisés en ce que** le composé d'appoint est un composé qui peut émettre de la lumière depuis l'état de triplet à température ambiante.

22. Solutions et formulations comprenant un ou plusieurs polymères ou blends selon une ou plusieurs des revendications 1 à 21 dans un ou plusieurs solvants.

23. Composés monomériques bifonctionnels de la formule (2) 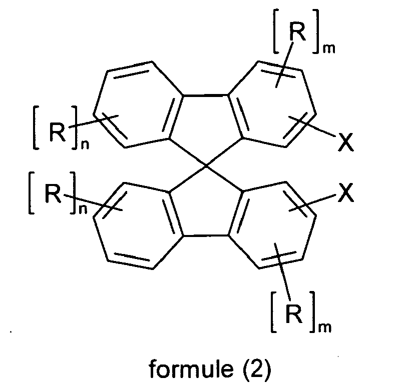 où R¹, n et m présentent la même signification que décrit selon la revendication 1, et en outre :
X est, pour chaque occurrence, de manière identique ou différente, un groupe choisi parmi le groupe constitué par CI, Br, I, O-tosylate, O-triflate, O-SO₂R¹, B(OR¹)₂ et Sn(R¹)₃, où R¹ présente la même signification que décrit selon la revendication 1, et où, en outre deux radicaux R¹ ou plus peuvent former un système de cycle les uns avec les autres ;
R est, pour chaque occurrence, de manière identique ou différente, CN, NO₂, OH, N(R¹)₂, Si(R¹)₃, B(R¹)₂, un groupe alkyle, alcoxy ou thioalcoxy en chaîne droite comportant de 1 à 40 atomes de C ou un groupe alkyle, alcoxy ou thioalcoxy ramifié ou cyclique comportant de 3 à 40 atomes de C, où, en plus, un ou plusieurs atomes de C non adjacents peuvent chacun être remplacés par -CR¹=CR¹-, -C≡C-, -NR¹-, -O-, -S-, -CO-O-, C=O, P(=O)R¹, SO, SO₂ ou -O-CO-O- et où, en plus, un ou plusieurs atomes de H peuvent être remplacés par fluor, un système de cycle aromatique ou hétéroaromatique comportant de 5 à 40 atomes de cycle aromatique, lequel peut également être substitué par un ou plusieurs radicaux R non aromatiques ; ou une combinaison de 2, 3, 4 ou 5 des groupes mentionnés ci avant ; en outre, deux des radicaux R ou plus afférents peuvent former un système de cycle aliphatique ou aromatique, mono- ou polycyclique les uns avec les autres ;
où le composé qui suit est exclu de l'invention : 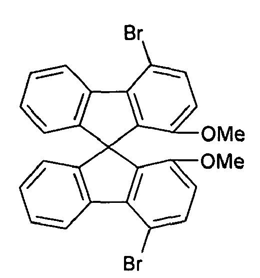 et en outre, étant entendu que les deux groupes X ne sont pas liés aux positions 2 et 2'.

24. Utilisation d'un polymère ou d'un blend selon une ou plusieurs des revendications 1 à 21 dans un dispositif électronique organique, en particulier en tant que matériau électroluminescent.

25. Dispositif électronique organique comportant une ou plusieurs couches actives, où au moins l'une de ces couches actives comprend un ou plusieurs polymères ou blends selon une ou plusieurs des revendications 1 à 21.

26. Dispositif électronique organique selon la revendication 25, **caractérisé en ce qu'**il s'agit d'une diode émettrice de lumière polymérique (PLED), d'un transistor à effet de champ organique (O-FET), d'un circuit intégré organique (O-IC), d'un transistor à film mince organique (O-TFT), d'une cellule solaire organique (O-SC), d'un dispositif à extinction de champ organique (O-FQD), d'un transistor émetteur de lumière organique (O-LET), d'une cellule électrochimique émettrice de lumière (LEC) ou d'une diode laser organique (O-laser).
